# EUROPÄISCHE PATENTSCHRIFT

(11) **EP 0 614 673 B1**
(45) Veröffentlichungstag und Bekanntmachung des Hinweises auf die Patenterteilung: **10.02.2010**
(21) Anmeldenummer: 94103011.6
(22) Anmeldetag: 01.03.1994
(51) Int. Cl.: A61M 1/00, A61M 27/00

(54) **Hydrocephalusventil**
Hydrocephalus valve
Valve pour le traitement de l'hydrocéphalie

(30) Priorität: 10.03.1993 DE 4307387; 19.01.1994 DE 4401422
(43) Veröffentlichungstag der Anmeldung: 14.09.1994
(73) Patentinhaber: Miethke, Christoph, 14469 Potsdam (DE)
(72) Erfinder: Miethke, Christoph, 14469 Potsdam (DE)
(74) Vertreter: Kaewert, Klaus

(56) Entgegenhaltungen:
- FR-A- 2 685 206
- US-A- 4 375 816
- US-A- 4 551 128
- US-A- 4 621 654
- US-A- 4 681 559

## Beschreibung

Die Erfindung betrifft ein Hydrocephalus-Ventil zum Druckausgleich im Schädel eines Hydrocephalus-Patienten.

Hydrocephalus-Patienten haben folgendes medizinisches Problem:

Das Gehirn ist im Schädel von einer besonderen Flüssigkeit - dem Liquor - umgeben. Dieser Liquor wird ständig produziert und im gleichen Maße resorbiert. Bei der Erkrankung des Hydrocephalus, auch Wasserkopf genannt, ist dieses Gleichgewicht gestört, und es wird mehr Flüssigkeit erzeugt als abgebaut wird. Da der Schädelinnenraum ein geschossenes Gefaßt darstellt, kommt es zu einer Volumenvergrößerung, beim Säugling können die Schädelnähte nicht zusammenwachsen, beim Erwachsenen steigt der Schädelinnendruck. Es gibt also einen Alters- und Kinderhydrocephalus.
Die Behandlung des Hydrocephalus erfolgte ursprünglich durch die bloße Ableitung des Liquors. Dies geschah durch die bloße Schlauchverbindung zwischen dem Schädel und einem großen venösen Blutgefäß oder durch eine entsprechende Verbindung des Schädels über einen Schlauch mit dem Bauchraum. Bald erkannte man jedoch, daß der Druck im Schädel einen bestimmten physiologischen Wert besitzen muß, wenn nicht wieder andere Komplikationen auftreten sollen.

Es ist bekannt, den Druck des Liquors im Schlauch mit Hilfe von Drosselventilen einzustellen. Derartige Drosselventile werden im Bereich des Kopfes unter der Haut implantiert. Die Ventile sollen sich bei einem bestimmten kritischen Druck öffnen und den Abfluß von Liquor gestatten. Dadurch wird einer Druckerhöhung entgegengewirkt. Über eine Leitung - ebenfalls unter der Haut implantiert - wird der Liquor in die obere Hohlvene oder die Bauchhöhle abgeleitet.

Die Funktion der bekannten Ventile ist jedoch unbefriedigend. Ein Vergleich der Messungen aller am Markt angebotenen Ventile zeigt, daß die Mehrheit der Ventile den Anforderungen bei weitem nicht genügt. Die Messungen ergeben teilweise Abweichungen von mehreren 100 %. Es kommt vor, daß bei Messungen nur jedes 5. Ventil den eingestellten Sollwert exakt einhält. Alle am Markt angebotenen Ventile nutzen das gleiche Funktionsprinzip: Der zu regelnde Druck wirkt auf eine federbelastete Fläche. Die resultierende Kraft aus der Druckkraft des Liquors und der Federkraft bestimmen das Öffnen oder Schließen eines Spaltes, durch den der Liquor fließen kann. Konstruktiv unterscheiden sich jedoch alle Ventile. Die Feder besteht aus Kunststoff oder Metall. Desgleichen variieren Form und Material des Schließkörpers. Die wichtigsten Ventile werden im folgenden kurz vorgestellt:

Das "Holter Minivalve" besteht aus einem elastischen Schlauch aus Silikonkautschuk. Parallel zu seiner Längsachse ist dieser Schlauch eingeschnitten. Dieser Schnitt öffnet sich zu einem Spalt, wenn der Innendruck ansteigt. Die Länge des Schnittes und die Wandstärke des Schlauches bestimmen die Öffnungscfiarakteristik.

Das "Heyer-Schulte Mini-LPV" ist ebenfalls ganz aus Silikonkautschuk gefertigt. Der Schließkörper wird durch eine Kugelkalotte gebildet, die gegen eine Öffnung gepreßt wird. Diese Kugelschale bildet gleichzeitig das elastische Element. Die Dicke der Schale bestimmt die Anpreßkraft und damit den Öffnungsdruck.

Das Ventil "CSF-Flow Control-Pudenz-Schulte Medical" hat das gleiche Funktionsprinzip wie das "Heyer-Schulte Mini LPV". Auch hier bildet der Verschlußkörper gleichzeitig das elastische Element.

Beim "Cordis Hakim Valve System" verschließt eine federbelastete Kugel die konische Öffnung des Ventiles. Übersteigt die Druckkraft der Flüssigkeit die Federkraft, wird die Kugel aus ihrem Sitz gehoben und gibt den Abfluß von Liquor frei. Die Feder kann sowohl als Blattfeder wie auch als Spiralfeder ausgeführt sein.

Es ist auch ein einstellbares Ventil unter der Bezeichnung "Valve programmable Sophy" bekannt, das die Verstellung des Öffnungsdruckes von außen ermöglicht, ohne die Haut dabei zu verletzen. Dies geschieht mit Hilfe eines Magneten, der über die Haut bewegt wird und mit dem im Inneren des Ventils ein Drehkörper verstellt wird. Am Drehkörper ist eine feine Blattfeder angebracht, die eine Kugel auf den Ventilsitz preßt. Durch die Drehung wird die Blattfeder kürzer oder länger eingespannt, wodurch die resultierende Federkraft verstellt wird.

Alle bekannten Ventile haben folgende Mängel:
sie verstopfen leicht,
der im Schädel erzeugte Druck weicht stark vom Sollwert ab,
der Druck ist stark vom Liquorfluß abhängig,
der Öffnungsdruck weicht stark vom Schließdruck ab,
der Druck ist stark von der Lage des Patienten abhängig.

Da eine Druck- und Flußmessung eines in einem Patienten implantierten Ventiles technisch schwierig ist, kann der Neurochirurg die Funktion des Ventiles nur schwer überprüfen und muß sich oft vom klinischen Bild des Patienten leiten lassen. Viele Ventile müssen wieder explantiert und durch neue ersetzt werden.

Aufgabe der Erfindung ist es, ein besseres Hydrocephalus-Ventil zu schaffen. Dabei geht die Erfindung von der Überlegung aus, den Liquorfluß zu regeln.

Zwar ist mit dem Orbis-Sigma-Ventil auch ein Hydrocephalus-Ventil bekannt, das auf Schwankungen der Druckwertdifferenz zwischen eroximalem und distalem Ventilabschnitt reagieren soll. Solche Druckdifferenzschwankungen sind unvermeidlich je nach Position des Patienten und abhängig von einem breiten Spektrum physiologischer Parameter (Lageveränderung, Husten, REM-Schlaf, physische und physische Belastung). Die Druckdifferenzschwankungen verursachen erhebliche Veränderungen im Liquorfluß.

Aus der US-Patentschrift 4681559 ist ein Ventilsystem bekannt, das ebenfalls als Parallelventil arbeitet. Eines der beiden parallel angeordneten Ventile spricht bei einem niedrigen Öffnungsdruck an, das andere bei einem höheren. Dem Niederdruckventil ist ein sehr enger Abflußkanal nachgeordnet, der dafür sorgen soll, daß auch vergleichsweise hohen Differenzdrücken, die aber niedriger sind als der Öffnungsdruck des anderen parallel angeordneten Ventils und die beispielswesie am Ventil anliegen, wenn der Patient sich zunehmend aufrichtet, einer Zunahme des Flows durch diese Ventilstufe entgegengewirkt wird. Die Reduzierung des Flows durch Verengung von abführenden Kanälen hat aber zwei wichtige Nachteile, die der Forderung nach optimaler Drainage entgegenwirken.

Erstens sind solche Kanäle nur wirksam, wenn sie lang sind und einen sehr engen Durchmesser haben. Die Realisierung eines engen Durchmessers erhöht aber signifikant die Gefahr der Verstopfung. Ein solches System ist also überaus anfällig. Damit werden klinisch beobachtete Probleme bei anderen Ventilsystemen, die ebenfalls mit engen Querschnittsflächen den Liqourabfluß durch das geöffnete Ventil regulieren wollen, nicht überwunden.

Zweitens kann auch durch eine solche Anordnung die schädliche Überdrainage in der stehenden Position des Patienten nicht verhindert werden. Zwar wird bei zunehmendem Differenzdruck am Ventil der Fowanstieg nicht so schnell zunehmen wie bei einfachen Differenzdruckventilen; ein erhöhter Flow gegenüber dem Flow in der reinen Liegendposition wird sich aber dennoch einstellen. Der höhere Differenzdruck treibt eine größere Flüssigkeitsmenge durch die Niederdruckseite des Ventilsystems und läßt damit eine erhöhte Flüssigkeitsmenge nach distal ablaufen. Die Überdrainage wird also bestenfalls zeitlich verschoben. Es ist bei der Betrachtung der Funktion von Hydrocephalus-Ventilen aber auch der Zustand einzubeziehen, bei dem beispielsweise in der Stehendposition des Patienten kein Liquor durch das System abzuleiten ist, sei es, weil die Liquorresorption und -produkten temporär im Gleichgewicht ist, sei es, weil aufgrund von Volumenverschiebungen im natürlichen Liquorsystem (z.B. nach distal in den Mark-Kanal) der Ventrikeldruck leicht negativ wird und ein kritischer Druck in den Ventrikeln des Patienten dann nicht erreicht wird. In diesen Fällen ist eine zusätzliche Entnahme von Liquor durch Abfluß durch das Ventil schädlich. Es können erhebliche Komplikationen entstehen.

Eine Verbesser gegenüber am etablierten einfach Differenzdrucksysstemen kann durch ein aus der US-PS 4681559 bekannten Ventilsystem als nicht erreicht werden.

Dagegen wird in der US-PS 4621654 erstmals eine Konstruktion beschrieben, die in Abhängigkeit von der Haltung des Patienten den Durchfluß derart steuert, daß in der stehenden Haltung des Patienten ein gegenüber der liegenden Patientenposition signifikant höherer Differenzdruck am Ventil anliegen muß, um eine Liquordrainage herbeizuführen. Allerdings ist bei diesem Vorschlag ein Kugelschalter parallel zu einer Ventilstufe angeordnet und nachgeschaltet muß eine zweite Vnetilstufe beim Liquorfluß passiert werden. Eine solche Anordnung bewirkt, daß entweder nur am unteren Ventil der Druck abfällt (Liegendposition des Patienten) oder aber zwei an den Ventilstufen anfallende Druckdifferenzen sich zu einem signifikant höheren Differenzdruck addieren. Ein solche Vorschlag kann nicht zu zufriedenstellenden Ergebnissen führten.

Der am Ventil anliegende Differendruck in der Stehendposition entspricht dem Öffnungsdruck des verschlossenen parallel angeordneten Ventils. Die Kalibrierung dieser Ventilstufe wird bestimmt durch die Größe des Patienten und den Öffnungsdruck des distal in Reihenschaltung angeordneten Ventil sfür die Liegendposition. Hierdurch kann nur ein vergleichsweise geringer Differenzdruck am Kugelschalter realisiert werden, wodurch das System dynamisch äußerst anfällig wird und in einzelnen Fällen bei Bewegungen des Patienten wie Gehen, Laufen, Hüpfen nahezu deaktiviert werden kann. Dem Vorteil, daß durch eine geringe anliegende Druckdifferenz die lagebedingte Schaltsicherheit des Ventils erhöht werden kann, steht also der Nachteil entgegen, daß das gesamte System bewegungsabhängig vollkommen deaktiviert werden kann..

Auch das aus der FR 2685206 bekannte Ventil trifft die Erfindung nicht. Das vorbekannte Ventil zeigt in der Fig. 5 einen Öffnung 37 als Zufluß. Die Öffnung 38 bildet in der Liegendfunktion den Abfluß.
Beim Wechsel von der Liegendstellung in eine Stehendstellung bewirkt die Druckerhöhung, daß sich die Membran 34 nach unten auswölbt und die Ventilteile 41 und 44 schließend aneinander zur Anlage kommen. Danach kann der Liquor nur noch über das Schlitzventil 46 entweichen. Bei diesem Vorschlag wird eine Ventilkammer mit einer Membran eingesetzt, durch deren Bewegung zwischen zwei Abflußleitungen hin und hergeschaltet wird.

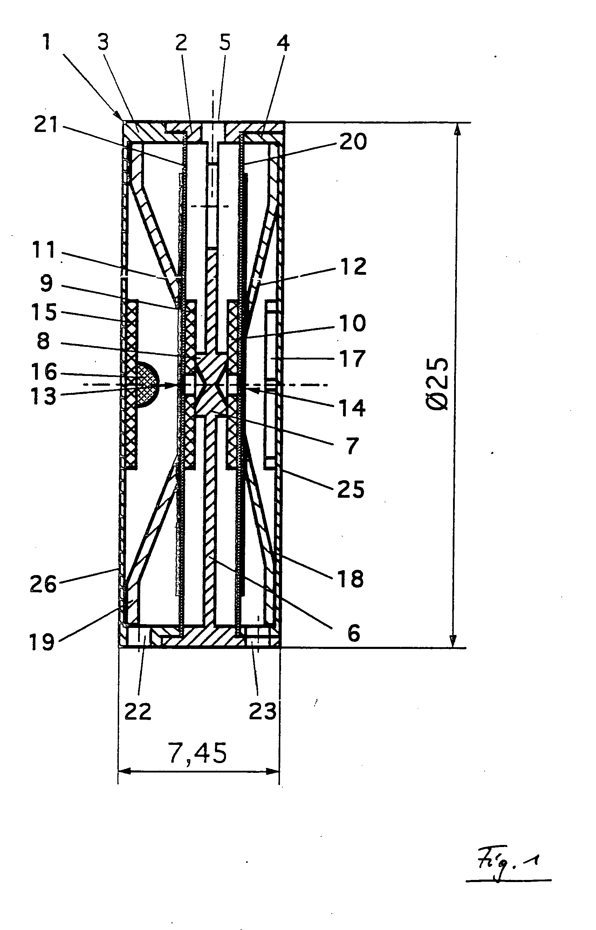

Soweit diese Druckschrift in der Fig. 1 ein Fließschema mit zwei Ventilen und einem weiteren, nachgeschalteten Kugelventil zeigt, ergibt sich, daß beide Ventile mit dem Kugelventil gesteuert werden. Das Kugelventil schließt in der Stehendposition und öffnet in der Liegendposistion. Die beiden von dem Kugelventil abhängigen Ventile können nur in der Liegendposition eine Wirkung entfalten.

Das Orbis-Sigma-Ventil soll dem durch drei verschiedene Ventilstellungen Rechnung tragen. In der ersten Höhenstufe soll das Ventil lediglich als Differentialdruckventil wirken. Hier ist der Differentialdruck niedrig und damit ein Unterdränagerisiko gering. Das Ventil bleibt geschlossen, solange der Differenzdruck unter dem Öffnungsdruck des Ventils liegt. Übersteigt der Differenzdruck den Öffnungsdruck, so bewegt sich eine Membrane im Ventil. Bei weiterem Anstieg der Druckdifferenz bewegt sich die Membrane weiter. Der Öffnungsquerschnitt wird jedoch im wesentlichen konstant gehalten. Infolgedessen wirkt das Ventil als Drossel. Es erhöht den Strömungswiderstand. Überdränagen werden infolgedessen in erheblichem Maße reduziert.

Bei noch weiterem Anstieg der Druckdifferenz vergrößert sich der Öffnungsquerschnitt der Membrane sprunghaft. Dadurch werden gefährliche Situationen vermieden.
Trotz der verschiedenen Ventileinstellungen beinhaltet das Orbis-Sigma-Ventil noch keine wesentliche Verbesserung gegenüber den herkömmlichen Ventilen.

Die Druckschriften U.S. 4.627.832 (1) und U.S. 4.776.838 (2) betreffen gleichfalls Ventile der erfindungsgemäßen Gattung. Auch bei diesen Ventilen werden unterschiedliche Zustände berücksichtigt, wie sie dem aufrechten und dem liegenden Patienten entsprechen. Eins der Ventile soll vier unterschiedliche Betriebszustände aufweisen, nämlich einen ersten, in dem das Ventil geschlossen ist, weil ein erster Ansprechdruck nicht erreicht wird. In einem zweiten Betriebszustand wird der erste Ansprechdruck erreicht, und das Ventil öffnet so weit, daß der betreffende eingangsseitige Liquordruck im wesentlichen aufrechterhalten wird. In einem dritten Betriebszustand wird eine im wesentlichen konstante Durchflußrate eingestellt. In einem vierten Betriebszustand hält das Ventil einen zweiten, höheren Druckwert des Liquors aufrecht. Dies wird mit einem einzigen Ventil erreicht und hat sich in der Praxis bisher nicht durchgesetzt.

In der DE-3020991 C2 (3) sind im Falle der Bauchwassersucht (Ascites) zwei parallele Teilventile in einer Ventilanordnung zum körperinternen Ableiten von Körperflüssigkeit vorgesehen. Mit dieser Ventilanordnung soll aber nur die Betriebssicherheit des Systems erhöht werden. Bei der Verstopfung eines Ventils arbeitet wenigstens das zweite weiter. Für Hydrocephalus-Ventile verspricht das keine Verbesserung gegenüber bekannten Ventilen.

Vor diesem Hintergrund ist in der Vergangenheit vorgeschlagen worden, ein als Regelventil ausgebildetes Hydrocephalus-Ventil zu verwenden, das unter der Haut im Bereich des Brustkorbes oder im Bauchbereich implantiert wird. Dort ist keine Miniaturisierung des Ventils erforderlich, weil ein ausreichendes Platzangebot gegeben ist.

Das Hydrocephalus-Ventil besitzt nach dem älteren Vorschlag eine Leitung zur Ableitung des Liquors, auf die eine Klemmeinrichtung wirkt. Die Klemmeinrichtung wird hydraulisch bedient, wobei als Hydraulikflüssigkeit der Liquor genutzt wird. Dies geschieht dadurch, daß als Klemmantrieb ein flexibles Behältnis verwendet wird, das mit dem Liquor beaufschlagt wird. Dazu dient eine Verbindungsleitung zwischen der den Liquor abführenden Leitung und dem Behältnis. Bei größer werdendem Druck bläht sich das Behältnis auf und drückt auf die Leitung. Bei geringer werdendem Liquordruck zieht sich das Behältnis zusammen, so daß die Klemmung nachläßt, die Leitung sich weiter öffnet.

Der Erfindung liegt die Aufgabe zugrunde, das Hydrocephalus-Ventil dem unterschiedlichen Leistungsbedarf besser anzupassen. Dabei geht die Erfindung von der Annahme aus, daß für das Öffnungsverhalten des Ventiles prinzipiell nur zwei Körperhaltungen maßgeblich sind. Das sind die vertikale und die horizontale Körperlage. Schräglagen sind nicht von großer Dauer und können vernachlässigt werden.

Diese Aufgabe ist durch die Merkmalkombination des Anspruchs 1 gelöst.

Beide Arbeitsbereiche werden durch den Liquordruck bestimmt. In einem Arbeitsbereich liegt der optimale Liquordruck bei etwa 12 cm Wassersäule, im anderen Arbeitsbereich bei 30 cm Wassersäule. Beide Werte bezeichnen den Differenzdruck gegenüber dem Flüssigkeitsdruck im Bauchraum. Der optimale Liquordruck kann je nach Patient variieren.

Nach der Erfindung besitzt das Ventil zwei Öffnungen, von denen die eine in der Liegendposition sich nach Erreichen des Bestimmungsdruckes öffnet und nach Erreichen des Bestimmungsdruckes für die Stehendposition die zweite Öffnung (Hochdrucköffnung) geöffnet und die erste Öffnung (Niederdrucköffnung) geschlossen wird.

Für die Ventilöffnung eignet sich besonders ein Doppelkammerventil, wobei die eine Kammer die Öffnung für die Liegendposition und die andere Kammer die Öffnung für die Stehendposition besitzt. Es ist von Vorteil, jeweils eine Kammerwand zugleich als bewegter Ventildeckel auszubilden. Dies geschieht in an sich bekannter Weise durch Verwendung einer Membran für die Kammerwand. Die Membran kann einen relativ geringen Verformungswiderstand besitzen. Die definierte Schließkraft wird dann mit Hilfe einer Rückstellfeder erreicht. Die Rückstellfeder ist vorzugsweise als Blattfeder ausgebildet.

Jede der eine Kammer bildenden Membrane ist mit einer Ventilöffnung versehen. Die Öffnung korrespondiert mit einer Schließfläche, die vorzugsweise ringförmig oder als Stopfen ausgebildet ist. Der Stopfen drückt sich in die Ventilöffnung, während die ringförmige Schließfläche die Ventilöffnung außen umgibt. Dazu ist zweckmäßig, die ringförmige Dichtfläche mit Dichtlippen zu versehen.

Wahlweise sind anstelle der Dichtlippen oder zusätzlich zu den Dichtlippen auch an der gegenüberliegenden Berührungsfläche der Membran nachgiebige Materialflächen vorgesehen, die ein schließendes Anlegen der Berührungsflächen erleichtern.

In der Zeichnung ist ein Ausführungsbeispiel der Erfindung dargestellt.

Mit 1 ist ein Ventilgehäuse eines Hydrocephalus-Ventiles bezeichnet. Das Gehäuse 1 besteht aus einem Mittelteil 2 und Außenteilen 3 und 4. Das Gehäuse ist rund, d. h. hat eine zylindrische Form, wobei der Zylinder im Ausführungsbeispiel einen Durchmesser von 25 mm besitzt und eine Höhe von 7,45 mm. Das Gehäuse besteht aus Titan. Titan ist ein verschleißfreier Werkstoff.

Die Einzelteile des Gehäuses bilden zentrische Ringe, die ineinandergeschoben werden können und sich mit Bund und Steg ineinander zentrieren.

Zu dem Gehäuseteil 3 gehört ein Deckel 26, zu dem Gehäuseteil 4 ein Deckel 25.
Der mittlere Gehäuseteil 2 besitzt eine Mittelwand 6, die mittig an jeder Seite jeweils eine ringförmige Verdickung 7 und 8 besitzt. Ferner ist im Mittelteil 2 eine Einlaßöffnung 5 vorgesehen. Im Anwendungsfall führt zur Einlaßöffnung 5 eine Schlauchleitung 30.

Das Ventil 1 ist im Bauchraum implantiert. Die Schlauchleitung führt vom Schädel des Patienten zu dem Ventil 1 und ist unter die Haut implantiert. Über die nicht dargestellte Schlauchleitung wird Liquor aus dem Schädel dem Ventil 1 zugeführt. Der Liquor verteilt sich beiderseits der Mittelwand 6 des Mittelteiles 2. Dies resultiert aus einer entsprechend gestalteten Einlaßöffnung 5.

Mit den ringförmigen Verdickungen 7 und 8 korrespondieren nachgiebige Kunststoffscheiben 9 und 10. Die Kunststoffscheiben 9 und 10 besitzen mittig Durchflußöffnungen 13 und 14. Die Durchtrittsöffnungen 13 und 14 liegen über den ringförmigen Verdickungen 7 und 8. Die Kunststoffscheiben dichten in der gezeigten Betriebsstellung schließend mit den Verdickungen 7 und 8.

Nach der Erfindung kann auch eine ungewollte Überdrainage verhindert werden. Dies wird nach der Erfindung durch die zusätzliche Verwendung eines Kugel/Konusverschlusses erreicht. Kugelventile haben bekanntermaßen gute Verschlußeigenschaft. Bei der Neukonstruktion ist das Prinzip des Kugel/Konusverschlusses invertiert. Hierdurch gelingt die Verknüpfung der Vorteile des Membranventils mit den Vorteilen des Kugelventils. Die Verwendung einer druckverstärkenden Membran ermöglicht die Realisierung eines größeren Strömungsquerschnittes durch den Verschluß des Ventils, was zu einer geringeren Hysterese führt. Der Flow-abhängige Druckanstieg bleibt vergleichsweise gering.

Die Kunststoffscheiben 9 und 10 sind an Kunststoffmembranen 21 und 20 befestigt, die als Scheiben zwischen das Mittelteil 2 und das äußere Teil 3 bzw. das Mittelteil und das äußere Teil 4 gebracht worden sind. Die Membranen 20 und 21 bestehen ihrerseits aus Kunststoff. Ihre Wandstärke ist relativ dünn ausgestaltet. Infolgedessen ist der Verformungswiderstand der Membranen 20 und 21 gering.
Den Membranen 20 und 21 ist durch Blattfedern 11 und 12 ein definiertes Verformungsverhalten gegeben. Die Blattfedern 11 und 12 können lose auf den Membranen 20 und 21 liegen. Sie können jedoch auch mit diesen z. B. verklebt sein.
Die Durchtrittsöffnungen 13 und 14 setzen sich durch die Membranen 21 und 20 und durch die Blattfedern 11 und 12 fort.

Im Ausführungsbeispiel sind zusätzlich zu den Blattfedern Stützen 18 und 10 vorgesehen. Die Stützen 18 und 19 sind tellerförmig ausgebildet. Sie fixieren die Blattfedern bei loser Anordnung in der gezeigten Ausgangsstellung.

Im Ausführungsbeispiel ist der Verformungsweg der Federn 11 und 12 ist begrenzt. Der Weg der Blattfeder 11 wird durch einen Stopfen 16 begrenzt, während der Weg der Blattfeder 12 durch eine Auflage 17 begrenzt wird. Der Stopfen 16 ist seinerseits aus nachgiebigem Kunststoffmaterial gefertigt und über eine Scheibe 15 gleichen Materials wie die Scheiben 9 und 10 an der Außenwand des Teiles 3 befestigt.
Durch Blattfedern 11 und 12, die an den Membranen 20 und 21 befestigt sind, können die Stützen 18 und 19 entbehrlich sein.

Das erfindungsgemäße Ventil 1 besitzt zwei Kammern, von denen die in der Zeichnung linke zum Teil 3 und die in der Zeichnung rechte zum Teil 4 gehört. Die linke Kammer ist als Niederdruckkammer ausgelegt, die rechte als Hochdruckkammer. Mit Niederdruck und Hochdruck sind im Ausführungsbeispiel relativ geringfügige Drücke bezeichnet, nämlich Drücke von etwa 12 bis 15 cm Wassersäule für die linke Kammer und etwa 30 cm Wassersäule für die rechte Kammer.

Im Ausführungsbeispiel ist die linke Kammer mit der Membrane 21 und der Blattfeder 11 und der Stütze 10 so ausgelegt, daß keine ausreichende Verformung eintritt, um einen Liquoreintritt in die linke Kammer zu ermöglichten, solange der Liquordruck keine Druckdifferenz von 12 cm Wassersäule gegenüber dem Druck im Bauchraum erreicht hat. Bei Erreichen dieser Druckdifferenz wird auf die Membrane bzw. die Blattfeder und die Stütze 19 ein Druck ausgeübt, der gleich dem Differenzdruck von 12 cm Wassersäule mal der Liquor-beaufschlagten Fläche ist. Druckbeaufschlagte Fläche ist dabei die Projektionsfläche in Öffnungsrichtung der Niederdruckventilkammer. D. h. nach Erreichen des resultierenden Druckes hebt die Membrane 21 mit der Scheibe 9 von der Verdickung ab, so daß Liquor zwischen der Scheibe 9 und der ringförmigen Verdickung hindurch in die Durchtrittsöffnung 13 und durch diese hindurch in die Niederdruckkammer strömen kann. Von dort kann der Liquor durch eine geeignete Öffnung in der Stütze 19 hindurch und durch eine Auslaßöffnung 22 hindurch austreten. Auf diesem Wege wird überschüssiger Liquor aus dem Schädel des Patienten abgeleitet. Der Ansprechdruck der Niederdruckkammer entspricht dem zulässigen Druck des Patienten in der Liegendstellung.

Im Ausführungsbeispiel.erhöht sich der durch die Niederdruckkammer gegebene Strömungswiderstand auch bei zunehmender Liquorproduktion nur geringfügig. D. h. die Federkennlinie der Membrane 21 der Blattfeder 11 und der Stütze 19 verläuft relativ flach. Im Ausführungsbeispiel erhöht sich der Liquordruck in der Niederdruckkammer in der Liegendstellung max. auf 15 cm Wassersäule.

Eine plötzliche Erhöhung des Liquordruckes auf 30 cm Wassersäule entsteht bei Änderung der Liegendposition in eine Stehendposition des Patienten. Dies führt dazu, daß die Blattfeder 11 sich an den Stopfen 16 anliegt, der die Durchtrittsöffnung 13 verschließt.

Auf der anderen Seite bewirkt der sich schlagartig erhöhende Liquordruck ein Nachgeben der Membrane 20 mit der Blattfeder 12 und der Stütze 18. Infolgedessen kann Liquor zwischen der Scheibe 10 und der ringförmigen Verdickung 7 hindurch in die Durchtrittsöffnung 14 treten. Die Durchtrittsöffnung 14 setzt sich durch die Blattfeder 12 fort, so daß der in die Hochdruckkammer eintretende Liquor über geeignete, nicht dargestellte Öffnungen in die Stütze 18 und eine Auslagsöffnung 23 entweichen kann. Die Betriebsweise der Hochdruckkammer ist die gleiche wie der der Niederdruckkammer, nur auf einem anderen Druckniveau. Außerdem wird die Durchtrittsöffnung (14) in der Membrane 20, der Blattfeder 12 auch durch Anlegen der Blattfeder 12 an die Scheibe 17 nicht verschlossen. Dazu ist die Scheibe 17 mit geeigneten Durchtrittskanälen versehen.

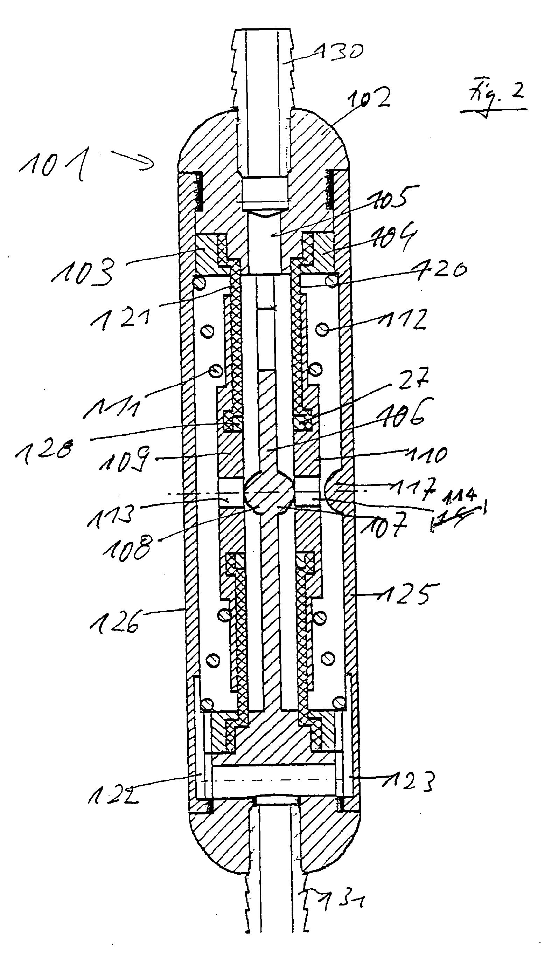

Mit 101 ist in Figur 2 ein Ventilgehäuse eines weiteren Hydrocephalus-Ventiles bezeichnet. Das Gehäuse 101 besteht aus einem Mittelteil 102 und Außenteilen 103 und 104. Das Gehäuse ist rund, d. h. hat wie in Figur 1 eine zylindrische, scheibenartige Form, wobei der Zylinder im Ausführungsbeispiel einen Durchmesser von 25 mm besitzt und eine Höhe von 7,45 mm. Das Gehäuse besteht aus Titan. Titan ist für den vorliegenden Anwendungsfall ein verschleißfreier Werkstoff.

Die Einzelteile des darüber hinaus außen abgerundeten Gehäuses bilden zentrische Ringe, die ineinander geschoben werden können und sich mit Bund und Steg ineinander zentrieren.

Zu dem Gehäuseteil 103 gehört ein Deckel 126, zu dem Gehäuseteil 104 ein Deckel 125. Der mittlere Gehäuseteil 102 besitzt eine Mittelwand 106, die mittig an jeder Seite eine halbkugelförmige Verdickung 107 und 108 aufweist. Ferner ist im Mittelteil 102 eine Einlaßöffnung 105 vorgesehen. Im Anwendungsfall führt zur Einlaßöffnung 105 eine Schlauchleitung 130.

Das Ventil 101 ist im Bauchraum implantiert. Die Schlauchleitung 130 führt vom Schädel des Patienten zu dem Ventil 101 und ist unter die Haut implantiert. Über die Schlauchleitung 130 wird Liquor aus dem Schädel dem Ventil 101 zugeführt. Der Liquor verteilt sich beiderseits der Mittelwand 106 des Mittelteils 102. Dies resultiert aus einer entsprechend gestalteten Einlaßöffnung 105.

Mit den ringförmigen Verdickungen 107 und 108 korrespondieren nachgiebige Scheiben 109 und 110 aus Titan. Die Scheiben 109 und 110 besitzen mittig Durchtrittsöffnungen 113 und 114. Die Durchtrittsöffnungen 113 und 114 liegen über den Verdickungen 107 und 108. Die Scheiben dichten mit den Verdickungen 107 und 108, wenn kein Liquor abzuführen ist.

Die Scheiben 109 und 110 sind an Kunststoffmembranen 121 und 120 befestigt, die als Scheiben zwischen das Mittelteil 102 und das äußere Teil 103 bzw. das Mittelteil und das äußere Teil 104 gebracht worden sind. Ihre Wandstärke ist relativ dünn ausgestaltet. Infolgedessen ist der Verformungswiderstand der Membranen 120 und 121 gering. Die Membranen sind am Außenrand zwischen den Teilen 103, 104, 105 geklemmt und am Innenrand zwischen den Schläuchen 109, 110 und Klemmringen 127, 128 gehalten.

Den Membranen 120 und 121 ist durch Spiralfedern 111 und 112 ein definiertes Verformungsverhalten gegeben. Die Federn 111 und 112 können lose auf den Membranen 120 und 121 liegen. Die Durchtrittsöffnungen 113 und 114 setzen sich durch die Membranen 121 und 120 und durch die Federn 111 und 112 fort.

Der Verformungsweg der Federn 111 und 112 ist begrenzt. Der Weg der Federn 112 wird durch einen Stopfen 117 begrenzt, während der Weg der Federn 111 durch eine Auflage am Deckel 126 begrenzt wird. Der Stopfen 117 ist mit dem Teil 125 einstückig. Bis auf die Membranen 120 und 121 aus Kunststoff, hier Silikon, sind alle Ventilteile im Ausführungsbeispiel aus Metall, hier Titan, gefertigt.

Das Ventil 101 besitzt zwei Kammern, von denen die in der Zeichnung linke zum Teil 103 und die in der Zeichnung rechte zum Teil 104 gehört. Die rechte Kammer ist als Niederdruckkammer ausgelegt, die linke als Hochdruckkammer. Mit Niederdruck und Hochdruck sind im Ausführungsbeispiel relativ geringfügige Drücke bezeichnet, nämlich Drücke von etwa 12 bis 15 cm Wassersäule für die rechte Kammer und etwa 30 cm Wassersäule für die linke Kammer.

Im Ausführungsbeispiel ist die rechte Kammer mit der Membrane 121 und der Feder 111 so ausgelegt, daß keine ausreichende Verformung eintritt, um einen Liquoreintritt in die rechte Kammer zu ermöglichen, solange der Liquordruck keine Druckdifferenz von 12 cm Wassersäule gegenüber dem Druck im Bauchraum erreicht hat. Bei Erreichen dieser Druckdifferenz wird auf die Membrane bzw. die Feder ein Druck ausgeübt, der gleich dem Differenzdruck von 12 cm Wassersäule mal der Liquorbeaufschlagten Fläche ist. Druckbeaufschlagte Fläche ist dabei die Projektionsfläche in Öffnungsrichtung der Niederdruckventilkammer. D. h. nach erreichen des resultierenden Druckes hebt die Membrane 120 mit der Scheibe 109 von der Verdickung ab, so daß Liquor zwischen der Scheibe 110 und der Verdickung 107 hindurch in die Durchtrittsöffnung 114 und durch diese hindurch in die Niederdruckkammer strömen kann. Von dort kann der Liquor durch eine Auslaßöffnung 123 hindurch austreten. Auf diesem Wege wird überschüssiger Liquor aus dem Schädel des Patienten in eine Leitung 131 in den Bauchraum abgeleitet. Der Ansprechdruck der Niederdruckkatmner entspricht dem zulässigen Druck des Patienten in der Liegendstellung.

Im Ausführungsbeispiel erhöht sich der durch die Niederdruckkammer gegebene Strömungswiderstand auch bei zunehmender Liquorproduktion nur geringfügig. D. h. die Federkennlinie der Membrane 120 und der Feder 112 verläuft relativ flach. Im Ausführungsbeispiel erhöht sich der Liquordruck in der Niederdruckkammer in der Liegendstellung max. auf 15 cm Wassersäule.

Eine plötzliche Erhöhung des Liquordruckes auf 30 cm Wassersäule entsteht bei Änderung der Liegendposition in eine Stehendposition des Patienten. Dies führt dazu, daß die Feder 111 sich an den Stopfen 117 anlegt, der die Durchtrittsöffnung 114 verschließt.

Auf der anderen Seite bewirkt der sich schlagartig erhöhende Liquordruck ein Nachgeben der Membrane 121 mit der Feder 111. Infolgedessen kann Liquor zwischen der Scheibe 109 und der ringförmigen Verdickung 108 hindurch in die Durchtrittsöffnung 113 treten. Die Durchtrittsöffnung 113 setzt sich fort, so daß der in die Hochdruckkammer eintretende Liquor über eine Austrittsöffnung 122 entweichen kann. Die Betriebsweise der Hochdruckkammer ist die gleiche wie die der Niederdruckkammer, nur auf einem anderen Druckniveau. Außerdem wird die Durchtrittsöffnung in der Membrane 21 nicht verschlossen.

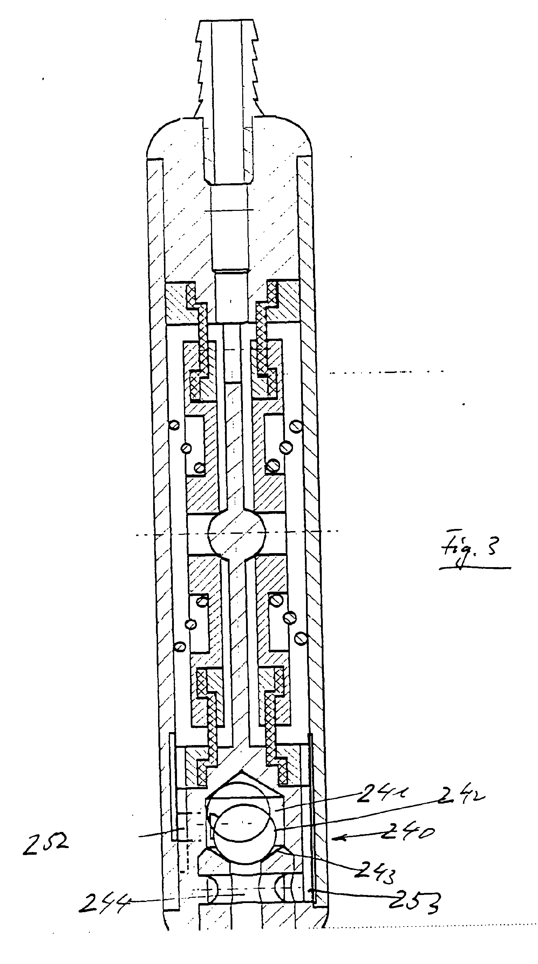

Figur 3 zeigt ein mit dem Ventil nach Figur 2 im wesentlichen gleiches Ventil mit zwei Auslaßöffnungen 252 und 253, wobei hinter der Auslaßöffnung 252 noch ein Kugel 242/Konus 243-Verschluß vorgesehen ist. Der zusätzliche Verschluß verhindert eine unerwünschte Leckage, z. B. der im Ausführungsbeispiel nach Figur 3 links angeordneten Niederdruckkammer. Der Verschluß ist dann in der Stehendposition geschlossen. Der Überdruck wird über die andere Kammer abgebaut. In der Liegendposition öffnet der Verschluß, weil die Kugel 242 aus dem Konus 243 in die Kammer 241 rollt.

Das Ventil könnte durch eine in der Zeichnung nach Figur 3 horizontale Anordnung auch eine andere Funktion erlangen. In dieser Anordnung könnte die rechte Kammer wie in Figur 2 die Niederdruckkammer sein, die linke die Hochdruckkammer. Der Verschluß würde dann in der Liegendstellung die Hochdruckseite schließen und in der Stehendposition öffnen.

Nach Figur 3 öffnet der dem Auslaß 252 gegenüberliegende Auslaß 253 in der Auslaß 244 der Kammer 241. Die Ausführung nach Figur 3 ist als invertierter Kugel/Konus-Verschluß zu bezeichnen.

## Patentansprüche

1. Hydrocephalus-Ventil zum Druckausgleich des Liquors im Schädel eines Hydrocephalus-Patienten; das im Brust- oder Bauiaum implantierbar ist und über eine unter der Haut des Patienten implantierten Schlauchleitung mit dessen Schädel verbunden ist, wobei das Ventil als Doppelkammerventil ausgebildet ist, das eine Niederdruckkammer und eine Hochdruckkammer ausweist,
**dadurch gekennzeichnet, daß**
die zwei Kammern des Doppelkammerventils durch eine Mittelwand (6) getrennt sind,
ein sich beiderseits der Mittelwand (6) öffnender Einlaß (5) für den Liquor vorgesehen ist, und jede Ventilkammer eine Membrane (20, 21) mit einer Feder (11,22) und einer Durchtrittsöffnung (13,14) aufweist.

2. Ventil nach Anspruch 1, **gekennzeichnet durch** einen am Wand (26) der Niederdruckkammer befestigte Stopfen (16) als Anschlag für die Membran (21) der Niederdruckkamer und als Verschlußelement von deren Durchtrittsöffnung (13) und/oder **durch** zwei, an den Membranen (20, 21) der beiden Kammernbefestigte ringförmige Stützen (9,10) als Anschlag für die Membranen (20, 21).

3. Ventil nach Anspruch 1 oder 2, **gekennzeichnet durch** eine ringförmige Verdickungen (7,8) in jeden Kammer an der zwischen beiden Kammern angeordneten Mittelwand (6) als Anschlag für die Membranen (20, 21) und als Verschlußelement für deren Durchtrittsöffnungen (13,14).

4. Ventil nach einem der Ansprüche 1 bis 3, **gekennzeichnet durch** die Anordnung eines zusätzlichen Kugel/Konus- Verschlusses (240) in Strömungsrichtung des Liquors hinter den Auslaßöffnungen (252, 253).

5. Ventil nach Anspruch 4, **gekennzeichnet durch** eine zusätzliche Kammer (241), die das Konus (243) des zusätzlichen Verschlusses (240) aufweist und die Kugel (242) des zusätzlienen Verschlusses (240) beweglich enthält, wobei die Kugel (242) in der Liegendstellung des Patienten aus dem Konus (243) rollt.

6. Ventil nach Anspruch 5, **dadurch gekennzeichnet, daß** die zusätzliche Kammer (241) mit der einen Auslaßöffnung (252) verbunden ist und die andere Auslaßöffnung (253) in den Kammerauslauf (244) mündet.

7. Ventil nach einem der Ansprüche 1 bis 6, **dadurch gekennzeichnet, daß** die Membranen aus Kunststoff und die übrige Teile aus Metall bestehen.

8. Ventil nach Anspruch 7, **dadurch gekennzeichnet, daß** als Kunststoff Silikon und als Metall Titan verwendet wird.

## Claims

1. Hydrocephalus valve for equalising the pressure of the liquor in the cranium of a hydrocephalus patient, the valve being implantable in the chest area or abdominal cavity and is connected to the patient's cranium with a tube line that is implanted under the skin of the patient, wherein the valve is designed as a double-chamber valve that possesses a low-pressure chamber and a high-pressure chamber,
**characterised in that**,
the two chambers of the double-chamber valve are separated by a centre wall (6),
an openable inlet (5) for the liquor is provided on both sides of the centre wall (6), and each valve chamber has a membrane (20,21) with a spring (11,22) and a passage opening (13,14).

2. Valve according to claim 1, **characterised by** a plug (16) fastened on the wall (26) of the low-pressure chamber as an arrestor for the membrane (21) of the low-pressure chamber and as a closure element for its passage opening (13) and/or by two circular supports (9,10) fastened on the membranes (20,21) of both of the chambers as an arrestor for the membranes (20,21).

3. Valve according to claim 1 or 2, **characterised by** a circular thickened part (7,8) in each chamber on the centre wall (6) disposed between both chambers as an arrester for the membranes (20,21) and as a closure element for its passage openings (13,14).

4. Valve according to one of claims 1 to 3, **characterised by** the placement of an additional ball/cone closure (240) in the direction of flow of the liquor behind the discharge openings (252,253).

5. Valve according to claim 4, **characterised by** an additional chamber (241) that possesses the cone (243) of the additional closure (240) and comprises the movable ball (242) of the additional closure (240), wherein the ball (242) rolls out of the cone (243) in the reclining position of the patient.

6. Valve according to claim 5, **characterised in that** the additional chamber (241) with which is connected one discharge opening (252) and the other discharge opening (253) opens out into the chamber outlet (244).

7. Valve according to one of claims 1 to 6, **characterised in that** the membranes consist of plastic and the other parts consist of metal.

8. Valve according to claim 7, **characterised in that** silicone is used as the plastic and titanium is used as the metal.

## Revendications

1. Valve pour le traitement de l'hydrocéphalie, pour l'équilibrage de pression du liquide céphalorachidien d'un patient souffrant d'hydrocéphalie, susceptible d'être implantée dans la Cavité thoracique ou la cavité abdominale et reliée à son crâne par l'intermédiaire d'une tuyauterie souple implantée sous la peau du patient, la valve étant réalisée sous forme de valve à chambre double, présentant une chambre basse pression et une chambre haute pression,
**caractérisée en ce que**
les deux chambres de la valve à chambre double sont séparées par une paroi médiane (6),
une admission (5), débouchant de part et d'autre de la paroi médiane (6), est prévue pour le liquide céphalorachidien, et chaque chambre de valve présente une membrane (20, 21) avec un ressort (11, 22) et une ouverture de passage (13, 14).

2. Valve selon la revendication 1, **caractérisée par** un bouchon (16), fixé sur la paroi (26) de la chambre basse pression, faisant office de butée pour la membrane (21) de la chambre basse pression et faisant office d'élément de fermeture de son ouverture de passage (13) et/ou par deux tubulures (9, 10) en forme d'anneau, fixées sur les membranes (20, 21) des deux chambres, faisant office de butée pour les membranes (20, 21),

3. Valve selon la revendication 1 ou 2, **caractérisée par** des épaississements (7, 8) dans chaque chambre, sur la paroi médiane (6) disposée entre les deux chambres, épaississements faisant office de butée pour les membranes (20, 21) et faisant office d'élément de fermeture pour leurs ouvertures de passage (13, 14).

4. Valve selon l'une des revendications 1 à 3, **caractérisée par** l'agencement d'une fermeture à bille/cône (240) supplémentaire, en aval des ouvertures d'échappement (252, 253) en observant dans la direction d'écoulement du liquide céphalorachidien.

5. valve selon la revendication 4, **caractérisée par** une chambre supplémentaire (241), présentant le cône (253) de la fermeture supplémentaire (240) et contenant, en état de mobilité, la bille (242) de la fermeture supplémentaire (240), la bille (242) roulant hors du cône (243) lorsque le patient est en position couchée.

6. Valve selon la revendication 5, **caractérisée en ce que** la chambre supplémentaire (241) est reliée à une première ouverture d'échappement (252), et l'autre ouverture d'échappement (253) débouche dans l'échappement de chambre (244).

7. Valve selon l'une des revendications 1 à 6, **caractérisée en ce que** la membrane est composée de matière synthétique et les autres pièces sont composées de métal.

8. Valve selon la revendication 7, **caractérisée en ce que** l'on utilise du silicone comme matière synthétique et du titane comme métal.
